Europäisches Patentamt

European Patent Office

Office européen des brevets

(11) Veröffentlichungsnummer : **0 331 021 B1**

# (12) EUROPÄISCHE PATENTSCHRIFT

(45) Veröffentlichungstag der Patentschrift :
06.05.92 Patentblatt 92/19

(51) Int. Cl.⁵ : **C07C 67/60, C07C 69/44**

(21) Anmeldenummer : **89103212.0**

(22) Anmeldetag : **23.02.89**

(54) **Verfahren zur Reinigung von Aldehyde, Acetale und/oder ungesättigte Verbindungen enthaltenden Carbonsäureestern.**

(30) Priorität : **27.02.88 DE 3806304**

(43) Veröffentlichungstag der Anmeldung :
06.09.89 Patentblatt 89/36

(45) Bekanntmachung des Hinweises auf die
Patenterteilung :
06.05.92 Patentblatt 92/19

(84) Benannte Vertragsstaaten :
**BE DE ES FR GB IT NL**

(56) Entgegenhaltungen :
**EP-A- 0 030 330**
**EP-A- 0 101 910**

(73) Patentinhaber : **BASF Aktiengesellschaft**
**Carl-Bosch-Strasse 38**
**W-6700 Ludwigshafen (DE)**

(72) Erfinder : **Frank, Gerhard, Dr.**
**Heidelberger Strasse 11**
**W-6945 Hirschberg (DE)**
Erfinder : **Lendle, Hubert, Dr.**
**Ruedigerstrasse 41**
**W-6700 Ludwigshafen (DE)**
Erfinder : **Seyfert, Wilfried, Dr.**
**Bachweg 8**
**W-6719 Weisenheim (DE)**
Erfinder : **Stops, Peter**
**Limburgstrasse 12**
**W-6701 Altrip (DE)**

EP 0 331 021 B1

## Beschreibung

Die Carbonylierung von Olefinen, d.h. die Umsetzung von beispielsweise Ethylen, Propylen oder Butylen mit Kohlenmonoxid und Alkanolen in Gegenwart von Carbonylkomplexen von Metallen der 8. Gruppe des periodischen Systems wird in der Technik im großen Maßstab zur Herstellung von Carbonsäureestern benutzt. Verwendet man als Ausgangsstoffe Diolefine, z.B. 1,3-Butadien so erhält man über die Zwischenstufe Pentensäureester Adipinsäuredimethylester ein wertvolles Ausgangsprodukt zur Herstellung von Faserrohstoffen. Da Kohlenmonoxid häufig in geringen Mengen Wasserstoff enthält oder durch Reaktion mit eingeschleppten Wasser sich Wasserstoff bildet, tritt neben der Carbonylierungsreaktion auch eine Hydroformylierungsreaktion ein. Diese führt zu Aldehyden und durch Umsetzung mit vorhandenen Alkoholen zu Acetalen. Des weiteren treten synthesebedingt ungesättigte Ketone, Tridekanone und Butendicarbonsäureester als Nebenprodukte in niedrigen Konzentrationen auf. Sofern die Siedepunkte der Acetale, Aldehyde oder ungesättigten Ketone sowie Butendicarbonsäureester mit den erzeugten Estern sehr eng beieinanderliegen, ist eine destillative Abtrennung dieser unerwünschten Nebenprodukte technisch außerordentlich aufwendig.

Die Abtrennung von Aldehyden, Acetalen und ungesättigten Verbindungen hat besondere Bedeutung bei der Herstellung von Adipinsäureestern, da die daraus hergestellte Adipinsäure dann für die Herstellung von Polymeren mit Faserqualität wenig geeignet ist. Darüber hinaus führen selbst geringe Mengen an Aldehyden, Acetalen und ungesättigten Verbindungen zu Verfärbungen der erzeugten Produkte, die unerwünscht sind.

Aus der europäischen Patentschrift 101 910 ist bereits ein Verfahren bekannt, bei dem man die Reinigung von Aldehyde, Acetale und/oder ungesättigte Verbindungen enthaltenden Carbonsäureestern durchführt, indem sie in einer Stufe in Gegenwart von mindestens ein Metall der 8. Nebengruppe des periodischen Systems enthaltenden sauren Ionenaustauschern oder Zeolithen bei erhöhter Temperatur mit Wasserstoff behandelt und die gebildeten Leicht- und/oder Höhersieder durch Destillation abtrennt. Dieses Verfahren hat den Nachteil, daß die Aktivität des Katalysators nachläßt und hierdurch der Gehalt an Verunreinigungen im Endprodukt ansteigt.

Es war deshalb die technische Aufgabe gestellt, ein Verfahren zur Verfügung zu stellen, das es ermöglicht, den Gehalt an verfärbenden Verunreinigungen zu erniedrigen und die Lebensdauer des Katalysators zu verbessern.

Diese Aufgabe wird gelöst in einem Verfahren zur Reinigung von Aldehyde, Acetale und/oder ungesättigte Verbindung enthaltenden Carbonsäureestern, die durch Umsetzen von olefinisch ungesättigten Verbindungen mit Kohlenmonoxid und Alkanolen erhalten worden sind, wobei man die Aldehyde, Acetale und/oder ungesättigte Verbindungen enthaltende Carbonsäureester mit sauren Mitteln und Wasserstoff in Gegenwart von mindestens einem Metall der 8. Nebengruppe des periodischen Systems behandelt und die gebildeten Leicht- und/oder Höhersieder destillativ abtrennt, dadurch gekennzeichnet, daß man die Aldehyde, Acetale und/oder ungesättigte Verbindungen enthaltenden Carbonsäureester

a) in einer ersten Stufe bei einer Temperatur von 20 bis 200 °C mit stark sauren Mitteln behandelt und
b) das so behandelte Gemisch in einer zweiten Stufe in Gegenwart von mindestens einem Metall der 8. Nebengruppe des periodischen Systems bei einer Temperatur von 50 bis 200 °C und unter einem Druck von 1 bis 50 bar hydriert.

Das neue Verfahren hat den Vorteil, daß der Anteil an verfärbenden Verunreinigungen erheblich vermindert wird und zudem die Lebensdauer des Katalysators verbessert wird.

Bevorzugte Carbonsäureester erhält man durch Carbonylierung von $C_2$-$C_{12}$-Monoolefinen, $C_4$-$C_{12}$-Diolefinen, $C_5$-$C_{12}$-Cycloalkenen oder $C_3$-$C_{12}$-Alkenmonocarbonsäure-$C_1$-$C_8$-Alkylestern. Die Carbonylierung erfolgt nach bekannten Verfahren durch Umsetzen mit Kohlenmonoxid und $C_1$-$C_8$-Alkanolen, insbesondere $C_1$-$C_4$-Alkanolen, z.B. bei Temperaturen von 100 bis 200 °C und unter Drücken von 50 bis 1000 bar in Gegenwart von Carbonylkomplexen von Metallen der 8. Nebengruppe des periodischen Systems, insbesondere von Kobalt- oder Rhodiumcarbonylkomplexen. Hieraus resultieren $C_3$-$C_{13}$-Alkanmonocarbonsäureester von Alkanolen mit 1 bis 8 Kohlenstoffatomen, $C_6$-$C_{14}$-Alkandicarbonsäureester von Alkanolen mit 1 bis 8 Kohlenstoffatomen oder Cycloalkancarbonsäureester mit 5 bis 12 Kohlenstoffatomen im Ring. Besonders bevorzugt sind gesättigte Alkanmono-und Dicarbonsäureester mit den obengenannten Kohlenstoffzahlen. So hergestellte Ester enthalten als Nebenprodukte Aldehyde und Acetale, wobei der Aldehydanteil die gleiche Kohlenstoffzahl aufweist wie die entsprechenden Carbonsäuren. Die Acetale enthalten darüber hinaus noch die jeweiligen Reste, die den mitverwendeten Alkanolen entsprechen. Zusätzlich sind noch ungesättigte Ketone oder ungesättigte Dicarbonsäureester, je nach Art der verwendeten Ausgangsstoffe enthalten. Der Gehalt an Aldehyden und Acetalen beträgt beträgt beispielsweise 0,1 bis 15 Gew.%. Geeignete Verfahren werden z.B. beschrieben in der US-PS 3 176 028 und DE-OS 1 618 156.

Besondere technische Bedeutung haben Adipinsäure-$C_1$-$C_4$-Alkylester erlangt, die durch Carbonylierung von Butadien oder Pentensäure-$C_1$-$C_4$-alkylestern mit Kohlenmonoxid und $C_1$-$C_4$-Alkanolen hergestellt worden

2

sind. Ein typisches Gemisch enthält beispielsweise neben Adipinsäureester 9 bis 14 Gew.% Methylglutarsäureester, 2 bis 5 Gew.% Ethylbernsteinsäureester, 0,01 bis 0,3 Gew.% 5-Formylvaleriansäureester sowie 0,01 bis 0,5 Gew.% 6,6-Dimethoxycarbonsäureester. Geeignete Verfahren werden z.B. beschrieben in der DE-PS 2 713 195.

Erfindungsgemäß wird das zu reinigende Gemisch in einer ersten Stufe bei einer Temperatur von 20 bis 200°C mit stark sauren Mitteln behandelt.

Geeignete stark saure Mittel sind z.B. Schwefelsäure, Phosphorsäure, Benzolsulfonsäure, Toluolsulfonsäure, bevorzugt sind stark saure Ionenaustauscher, z.B. vernetztes Polystyrol mit Sulfonsäuregruppen, ferner Aluminium- oder Borsilikatzeolithe in der sauren Form. Bei den stark sauren Ionenaustauschern wie Aluminium- oder Borsilikatzeolithen wendet man vorteilhaft 0,01 bis 0,1 kg, je kg zugeführtem Ausgangsgemisch an, während man für die restlichen der genannten sauren Mittel 1 bis 50 Gew.%, bezogen auf die Menge der Acetale einsetzt. Die Reaktionsdauer beträgt im allgemeinen 0,1 bis 4 Stunden. Vorteilhaft hält man eine Temperatur von 60 bis 140°C an. Bei der Verwendung von sauren Ionenaustauschern auf Basis vernetzten Polystyrols sollte jedoch eine Temperatur von 130°C nicht überschritten werden.

Ferner hat es sich bewährt, wenn man die bei der Behandlung entstehenden Alkohole durch Behandeln mit in Inertgasen, z.B. Stickstoff, strippt. Ferner hat es sich bewährt, Wasser in geringen Mengen, z.B. in einer Menge von 0,5 bis 2,0 Mol/Mol Acetal mitzuverwenden.

Das so erhaltene Gemisch wird erfindungsgemäß in einer getrennten zweiten Stufe in Gegenwart von mindestens einem Metall der 8. Nebengruppe des periodischen Systems bei einer Temperatur von 50 bis 200°C und unter einem Druck von 1 bis 50 bar hydriert.

Geeignete Metalle sind beispielsweise Kobalt, Nickel, Paladium oder Rhodium. Besonders bevorzugt sind Kobalt oder Paladium. Die Katalysatoren können als Voll- oder Trägerkatalysatoren angewandt werden. Bei Vollkatalysatoren, wie Kobalt oder Nickel, hat sich die Modifizierung mit Promotoren, z.B. Mangan in einer Menge von 2 bis 5 Gew.%, bezogen auf Kobalt oder Nickel und/oder die Modifizierung mit Phosphorsäure z.B. einem Gehalt an Phosphorsäure berechnet als $P_2O_5$ von 1 bis 4 Gew.%, bezogen auf Kobalt und/oder Nickel, bewährt. Sofern Trägerkatalysatoren angewandt werden, eignen sich als Träger beispielsweise Siliziumdioxyd, Aluminiumoxyd, Aktivkohle oder Silikate.

Die Trägerkatalysatoren haben vorteilhaft einen Gehalt von 0,2 bis 5 Gew.%, insbesondere 0,2 bis 2 Gew.% der genannten Metalle.

Die Hydrierung wird bei einer Temperatur von 50 bis 200°C vorteilhaft bei einer Temperatur von 100 bis 170°C und unter einem Druck von 1 bis 50 bar, vorzugsweise 10 bis 30 bar, durchgeführt. Die Verweilzeit beträgt vorzugsweise 0,3 bis 3 Stunden.

Aus dem so erhaltenen hydrierten Gemisch werden die Hochsieder und Leichtsieder durch Destillation von den Carbonsäureestern abgetrennt.

Vorteilhaft führt man die Destillation durch indem man die Leicht- und Höhersieder von den Carbonsäureestern durch Destillation in zwei hintereinandergeschalteten Kolonnen abtrennt, wobei man in der ersten Kolonne die Höhersieder als Sumpfprodukt abtrennt und das Gemisch aus Leichtsiedern und Carbonsäureestern am Kopf der Kolonne entnimmt und aus diesem Gemisch in der zweiten Kolonne die Leichtsieder als Kopfprodukt abtrennt und die Carbonsäureester im unteren Teil der Kolonne entnimmt. In der Regel führt man das zu trennende Gemisch jeweils im zweiten Drittel der Kolonnen zu. Die Entnahme der reinen Carbonsäureester in der zweiten Kolonne erfolgt zweckmäßig im Sumpf, insbesondere 1 bis 5 theoretische Böden über dem Sumpf.

Zweckmäßig verwendet man Kolonnen mit Füllkörpern , Glockenböden, Siebböden, insbesondere mit strukturierten Packungen, die einen Druckabfall je Boden von weniger als 2,5 mbar gewährleisten. Vorzugsweise haben die Kolonnen 40 bis 120 theoretische Böden. Es hat sich darüber hinaus bewährt, ein Rücklaufverhältnis von 1 bis 10 einzuhalten.

Nach einer modifizierten Arbeitsweise verfährt man so, daß man die Leicht- und Höhersieder enthaltenden Carbonsäureester im mittleren Teil einer ersten Kolonne zuführt, am Kopf der Kolonne Leichtsieder entnimmt, im Sumpf der Kolonne Höhersieder abtrennt, im unteren Drittel der Kolonne einen Teil des dampfförmigen Gemisches entnimmt und in das untere Ende einer zweiten Kolonne leitet, im mittleren Teil der zweiten Kolonne reine Carbonsäureester entnimmt und das dampfförmige Gemisch aus dem Kopf der zweiten Kolonne wieder dem oberen Dritten der ersten Kolonne zuführt.

Nach einer besonders vorteilhaften Arbeitsweise verfährt man so, daß man die Leicht- und Höhersieder von den Carbonsäureestern durch Destillation in einer Kolonne abtrennt, wobei man das zu trennende Gemisch in der unteren Hälfte der Kolonne zuführt, in der oberen Hälfte der Kolonne Carbonsäureester seitlich entnimmt und Leichtsieder am Kopf der Kolonne und Höhersieder im Sumpf der Kolonne abzieht.

Vorzugsweise verwendet man eine Kolonne mit Einbauten wie oben beschrieben, die 60 bis 150 theoretische Böden hat. Das zu trennende Gemisch führt man vorteilhaft im zweiten Viertel (von unten gerechnet) der Kolonne zu. Reine Carbonsäureester werden in der oberen Hälfte z.B. aus dem dritten Viertel (von unten gerechnet) seitlich entnommen. Ein Rücklaufverhältnis, bezogen auf die Seitenabzugsmenge, von 2 bis 10 hat sich bewährt.

Nach einer modifizierten Arbeitsweise verfährt man so, daß man die Leicht- und Höhersieder von den Carbonsäureestern durch Destillation in einer Kolonne abtrennt, wobei man das zu trennende Gemisch dem zweiten Viertel der Kolonne zuführt, im dritten Viertel der Kolonne Carbonsäureester seitlich entnimmt und Leichtsieder am Kopf der Kolonne und Höhersieder im Sumpf der Kolonne abzieht und wobei die Kolonne in den mittleren zwei Vierteln der Länge nach in zwei Zonen geteilt ist, mit der Maßgabe, daß der Zulauf in die erste Zone erfolgt und in der zweiten Zone Carbonsäureester entommen wurde.

Die erfindungsgemäß gereinigten Ester eignen sich als Lösungsmittel. Adipinsäureester werden zu Adipinsäure verseift, das ein Ausgangsprodukt zur Herstellung von Polykondensaten, wie Polyamid-6,6 ist.

Das Verfahren nach der Erfindung sei an folgenden Beispielen veranschaulicht.

Vergleichsbeispiel 1

In einen Rohrreaktor von 40 Liter Inhalt, der mit einem starksauren Ionenaustauscher auf Basis Polystyrol mit Sulfonsäuregruppen enthaltend 5 g Paladium je Liter Ionenaustauscher beschickt ist, werden stündlich 13 kg Adipinsäuredimethylester, der 0,15 Gew.% 6,6-Dimethoxycapronsäuremethylester, 0,03 Gew.% Butendicarbonsäuredimethylester, 0,03 Gew.% Tridecanon sowie 0,2 Gew.% 2-Methylglutarsäuredimethylester sowie Spuren an 5-Formylvaleriansäuremethylester enthält und eine UV-Zahl von 19 000 hat, zusammen mit 50 N Liter Wasserstoff zudosiert. Das Gemisch wird bei einer Temperatur von 110°C und unter einem Druck von 30 bar hydriert. Der Reaktionsaustrag wird entspannt und rektifiziert. Das so erhaltene Reinprodukt enthält 99,74 Gew.% Adipinsäuredimethylester und hat eine UV-Zahl von 1 100. Nach einer Betriebsdauer von 400 Stunden steigt die UV-Zahl auf 1 310.

Beispiel 1

In einen Rohrreaktor von 40 Liter Inhalt, der mit Y-Zeolith in der H$^+$-Form beschickt ist, werden stündlich 13 kg Adipinsäuredimethylester der 0,15 Gew.% 6,6-Dimethoxycapronsäuremethylester, 0,03 Gew.% Butendicarbonsäuredimethylester, 0,03 Gew.% Tridecanon, 0,2 Gew.% Methylglutarsäuredimethylester sowie Spuren an Formylvaleriansäuremethylester enthält und eine UV-Zahl von 19 000 hat, zudosiert und bei einer Temperatur von 120°C behandelt.

In einer zweiten Stufe wird der so erhaltene Reaktionsaustrag in einem zweiten Reaktionsrohr von 40 Liter Inhalt, das mit Aluminiumoxid mit einem Gehalt von 0,7 Gew.% Paladium beschickt ist, zusammen mit 50 N Liter Wasserstoff zugeführt und die Hydrierung bei einer Temperatur von 135°C unter einem Druck von 30 bar durchgeführt. Der erhaltene Reaktionsaustrag wird entspannt und durch Destillation von Leicht- und Hochsieden befreit. Nach der Rektifikation erhält man 99,8 gew.%igen Adipinsäuredimethylester mit einer UV-Zahl von 900. Nach einer Betriebszeit von 400 Stunden ist eine Desaktivierung des Hydrierkatalysators nicht erkennbar.

Beispiel 2

Man behandelt wie in Beispiel 1 beschrieben Verunreinigungen enthaltenden Adipinsäuredimethylester mit y-Zeolith in der H-Form.

In ein über Doppelmantel beheizbares Rohr (innerer Durchmesser 2,5 cm, Höhe 15 cm) wurden 80 ml (=130 g) eines bei 330°C mit H$_2$ reduzierten Cobaltkatalysators in Splittform (1-2 mm) eingebracht. Vor der Reduktion des oxidischen Katalysators lag die Zusammensetzung des Katalysators bei 90,40 % CoO, 5,04 % Mn$_2$O$_3$, 2,22 % P$_2$O$_5$ und 1,40 Na$_2$O. Der Katalysator hatte einen Reduktionsgrad von 80 % bezogen auf Cobalt und Mangan. Bei 120°C und 20 bar H$_2$ wurden stündlich 300 ml rohes Dimethyladipat wie in Beispiel 1 vorbehandelt über diesen Katalysator geleitet. Nach fraktionierter Destillation des Austrags der reduktiven Nachbehandlung konnte eine Hauptfraktion (98 %) mit einer UV-Zahl von 300 erzielt werden.

Die UV-Zahl verursachenden Komponenten befanden sich zum weit überwiegenden Anteil im Vorlauf.

Nach 1000 Stunden war noch keine Abnahme der Katalysatoraktivität erkennbar.

Vergleichsbeispiel 2

In die in Beispiel 2 beschriebene Vorrichtung wurden 80 ml eines 0,5 % Palladium enthaltenden Zeoliths eingebracht. Nach der Aktivierung des Katalysators mit Wasserstoff wurden bei 120°C und 20 bar H$_2$ stündlich 300 ml des acetalhaltigen rohen Dimethyladipats zugeführt.

Es wurde von Adipinsäuredimethylester einer UV-Zahl von 12498 ausgegangen. Nach vergleichbarer fraktionierter Destillation des Hydrieraustrags lag die UV-Zahl bei 1200. Die UV-Zahl verursachenden Komponenten befanden sich vorwiegend im Nachlauf der Destillation.

Nach 48 Stunden läßt die Aktivität des Katalysators erheblich nach.

Vergleichsbeispiel 3

Nach saurer und hydrierender Behandlung eines Adipinsäuredimethylesters, wie in Beispiel 1 beschrieben, wurde das Reaktionsgemisch zweistufig aufdestilliert. Der Zulauf erfolgte in dem unteren Teil der ersten Kolonne, die mit 70 theoretischen Böden ausgestattet war. Dort wurden die leichtsiedenden Verunreinigungen bei einem Rücklaufverhältnis von 10 über Kopf abgetrennt. Der Hauptstrom, der im Sumpf dieser Vakuumkolonne mit 160°C anfiel, wurde anschließend erneut destilliert, indem er den unteren Teil einer Vakuumkolonne (60 mbar) mit 90 theoretischen Böden zugeführt wurde. Bei einem Rücklaufverhältnis von 2,5 konnte über Kopf ein gereinigtes Estergemisch mit einer UV-Zahl von 1250 erhalten werden.

Beispiel 3

Führt man die destillative Aufarbeitung des sauer und hydrierend behandelten Adipinsäuredimethylesters (s. Beispiel 1) einstufig durch, indem man das Reaktionsgemisch im unteren Teil einer Kolonne (100 theor. Böden) zuführt und das Wertprodukt in der oberen Hälfte über Seite abzieht, fällt dieses bei einem Rücklaufverhältnis von 4, bezogen auf den Seitenabzug, mit einer UV-Zahl von 900 an. Bei der unter Vakuum durchgeführten Destillation stellt sich eine Sumpftemperatur von 165 °C ein.

**Patentansprüche**

1. Verfahren zur Reinigung von Aldehyde, Acetale und/oder ungesättigte Verbindungen enthaltenden Carbonsäureestern, die durch Umsetzen von olefinisch ungesättigten Verbindungen mit Kohlenmonoxyd und Alkanolen erhalten worden sind, wobei man die Aldehyde, Acetale und/oder ungesättigte Verbindungen enthaltende Carbonsäureester mit sauren Mitteln und Wasserstoff in Gegenwart von mindestens einem Metall der 8. Nebengruppe des periodischen Systems bei erhöhter Temperatur behandelt und die gebildeten Leicht- und/oder Höhersieder destillativ abtrennt, dadurch gekennzeichnet, daß man die Aldehyde, Acetale und/oder ungesättigte Verbindungen enthaltenden Carbonsäureester
   a) in einer ersten Stufe bei einer Temperatur von 20 bis 200 °C mit stark sauren Mitteln behandelt und
   b) das so behandelte Gemisch in einer zweiten Stufe in Gegenwart von mindestens einem Metall der 8. Nebengruppe des periodischen Systems bei einer Temperatur von 50 bis 200°C und unter einem Druck von 1 bis 50 bar hydriert.

2. Verfahren nach Anspruch 1, dadurch gekennzeichnet, daß man in der ersten Stufe Alkanole durch Strippen mit Inertgasen entfernt.

3. Verfahren nach den Ansprüchen 1 und 2, dadurch gekennzeichnet, daß man in der ersten Stufe eine Temperatur von 60 bis 140°C einhält.

4. Verfahren nach den Ansprüchen 1 bis 3, dadurch gekennzeichnet, daß man in der zweiten Stufe eine Temperatur von 100 bis 170°C einhält.

5. Verfahren nach den Ansprüchen 1 bis 4, dadurch gekennzeichnet, daß man die Leicht- und Höhersieder von den Carbonsäureestern durch Destillation in zwei hintereinandergeschalteten Kolonnen abtrennt, wobei man in der ersten Kolonne die Höhersieder als Sumpfprodukt abtrennt und das Gemisch aus Leichtsiedern und Carbonsäureestern am Kopf der Kolonne entnimmt und aus diesem Gemisch in der zweiten Kolonne die Leichtsieder als Kopfprodukt abtrennt und die Carbonsäureester im unteren Teil der Kolonne entnimmt.

6. Verfahren nach den Ansprüchen 1 bis 4, dadurch gekennzeichnet, daß man die Leicht- und Höhersieder enthaltenden Carbonsäureester im mittleren Teil einer ersten Kolonne zuführt, am Kopf der Kolonne Leichtsieder entnimmt, im Sumpf der Kolonne Höhersieder abtrennt, im unteren Drittel der Kolonne einen Teil des dampfförmigen Gemisches entnimmt und in das untere Ende einer zweiten Kolonne leitet, im mittleren Teil der zweiten Kolonne reine Carbonsäureester entnimmt und das dampfförmige Gemisch aus dem Kopf der zweiten Kolonne wieder dem oberen Dritten der ersten Kolonne zuführt.

7. Verfahren nach den Ansprüchen 1 bis 4, dadurch gekennzeichnet, daß man die Leicht- und Höhersieder von den Carbonsäureestern durch Destillation in einer Kolonne abtrennt, wobei man das zu trennende Gemisch in der unteren Hälfte der Kolonne zuführt, in der oberen Hälfte der Kolonne Carbonsäureester seitlich entnimmt und Leichtsieder am Kopf der Kolonne und Höhersieder im Sumpf der Kolonne abzieht.

8. Verfahren nach den Ansprüchen 1 bis 4, dadurch gekennzeichnet, daß man die Leicht- und Höhersieder von den Carbonsäureestern durch Destillation in einer Kolonne abtrennt, wobei man das zu trennende Gemisch dem zweiten Viertel der Kolonne zuführt, im dritten Viertel der Kolonne Carbonsäureester seitlich entnimmt und Leichtsieder am Kopf der Kolonne und Höhersieder im Sumpf der Kolonne abzieht und wobei die Kolonne in den mittleren zwei Vierteln der Länge nach in zwei Zonen geteilt ist, mit der Maßgabe, daß der Zulauf in die erste Zone erfolgt und in der zweiten Zone Carbonsäureester entommen wurde.

## Claims

1. A process for purifying a carboxylic ester which has been obtained by reacting an olefinically unsaturated compound with carbon monoxide and an alkanol and contains an aldehyde, an acetal or an unsaturated compound by treating said ester with an acidic agent and hydrogen at elevated temperatures in the presence of one or more metals of sub-group VIII of the periodic table and removing the low and/or high boilers formed by distillation, which comprises

a) treating the carboxylic ester which contains an aldehyde, acetal or unsaturated compound in a first stage at from 20 to 200°C with a strongly acidic agent and

b) hydrogenating the mixture thus treated in a second stage at from 50 to 200°C under a pressure of from 1 to 50 bar in the presence of one or more metals of sub-group VIII of the periodic table.

2. A process as claimed in claim 1, wherein alkanol is removed in the first stage by stripping with an inert gas.

3. A process as claimed in either of claims 1 and 2, wherein the first stage is maintained at from 60 to 140°C.

4. A process as claimed in any of claims 1 to 3, wherein the second stage is maintained at from 100 to 170°C.

5. A process as claimed in any of claims 1 to 4, wherein the low and high boilers are separated from the carboxylic ester by distillation in two columns connected in series, the high boilers being removed in the first column as bottom product and the mixture of low boilers and carboxylic ester being withdrawn at the top of the column and, in the second column, the low boilers being separated from this mixture as heads and the carboxylic ester being withdrawn in the bottom part of the column.

6. A process as claimed in any of claims 1 to 4, wherein the carboxylic ester which contains low and high boilers is introduced into the central part of the first column, low boilers are removed at the head of the column, high boilers are separated off at the base of the column, a portion of the vaporous mixture is removed in the lower third of the column and passed into the lower end of the second column, pure carboxylic ester is removed in the central portion of the second column, and the vaporous mixture is recycled from the top of the second column back into the upper third portion of the first column.

7. A process as claimed in any of claims 1 to 4, wherein the low and high boilers are separated from the carboxylic ester by distillation in a column, the mixture to be separated being introduced into the bottom half of the column, carboxylic ester being removed sideways in the upper half of the column, and low boilers being removed at the top of the column and higher boilers at the base of the column.

8. A process as claimed in any of claims 1 to 4, wherein the low and high boilers are separated from the carboxylic ester by distillation in a column, the mixture to be separated being introduced into the second quarter of the column, carboxylic ester being removed sideways in the third quarter of the column and low boilers being removed at the head of the column and high boilers at the base of the column, the column having been split lengthwise in two zones in the middle two thirds, with the proviso that the feed is into the first zone and carboxylic ester is removed from the second zone.

## Revendications

1. Procédé de purifiration d'esters d'acides carboxyliques qui contiennent des aldéhydes, des acétals et/ou des composés insaturés et qui ont été obtenus par réaction de composés à insaturation oléfinique avec du monoxyde de carbone et des alcanols, les esters d'acides carboxyliques qui contiennent des aldéhydes, des acétals et/ou des composés insaturés étant traités, à température élevée, par des agents acides et de l'hydrogène en présence d'au moins un métal du groupe VIII de la classification périodique et les produits de bas point d'ébullition et/ou de point d'ébullition plus élevé qui sont formés étant séparés par distillation, caractérisé en ce que

a) dans une première étape, on traite les esters d'acides carboxyliques contenant des aldéhydes, des acétals et/ou des composés insaturés, à une température de 20 à 200°C, par des agents fortement acides et

b) dans une seconde étape, on hydrogène le mélange ainsi traité, en présence d'au moins un métal du groupe VIII de la classification périodique, à une température de 50 à 200°C et sous une pression de 1 à 50 bar.

2. Procédé selon la revendication 1, caractérisé en ce que, dans la première étape, on élimine les alcanols par entraînement par des gaz inertes.

3. Procédé selon la revendication 1 ou 2, caractérisé en ce que dans la première étape, on maintient une température de 60 à 140°C.

4. Procédé selon l'une quelconque des revendications 1 à 3, caractérisé en ce que dans la seconde étape, on maintient une température de 100 à 170°C.

5. Procédé selon l'une quelconque des revendications 1 à 4, caractérisé en ce qu'on sépare des esters d'acides carboxyliques les produits de bas point d'ébullition et/ou de point d'ébullition plus élevé dans deux colonnes montées l'une à la suite de l'autre, les produits de point d'ébullition plus élevé étant séparés en tant que produit de bas de colonne dans la première colonne et le mélange de produits de bas point d'ébullition et d'esters d'acides carboxyliques étant prélevé en tête de la colonne, les produits de bas point d'ébullition étant séparés de ce mélange en tant que produit de tête dans la seconde colonne et les esters d'acides carboxyliques étant prélevés dans la partie inférieure de la colonne.

6. Procédé selon l'une quelconque des revendications 1 à 4, caractérisé en ce qu'on introduit les esters d'acides carboxyliques contenant des produits de bas point d'ébullition et de point d'ébullition plus élevé dans la partie moyenne d'une première colonne, on prélève des produits de bas point d'ébullition en tête de la colonne, on sépare des produits de point d'ébullition plus élevé au bas de la colonne, on prélève une partie du mélange à l'état de vapeur dans le tiers inférieur de la colonne et on l'envoie dans la partie inférieure d'une seconde colonne, on prélève des esters d'acides carboxyliques purs dans la partie aryenne de la seconde colonne et on renvoie dans le tiers supérieur de la première colonne le mélange à l'état de vapeur provenant de la tête de la seconde colonne.

7. Procédé selon l'une quelconque des revendications 1 à 4, caractérisé en ce qu'on sépare les produits de bas point d'ébullition et de point d'ébullition plus élevé des esters d'acides carboxyliques par distillation dans une colonne, le mélange à séparer étant introduit dans la moitié inférieure de la colonne, les esters d'acides carboxyliques étant prélevés latéralement dans la moitié supérieure de la colonne, les produit de bas point d'ébullition étant extraits en tête de la colonne et les produits de point d'ébullition plus élevé étant extraits au bas de la colonne.

8. Procédé selon l'une quelconque des revendications 1 à 4, caractérisé en ce qu'on sépare les produits de bas point d'ébullition et de point d'ébullition plus élevé des esters d'acides carboxyliques par distillation dans une colonne, le mélange à séparer étant introduit dans le deuxième quart de la colonne, les esters d'acides carboxyliques étant prélevés latéralement dans le troisième quart de la colonne, les produits de bas point d'ébullition étant extraits en tête de la colonne et les produits de point d'ébullition plus élevé étant extraits au bas de la colonne, la colonne dans les deux quarts médians étant divisée en longueur en deux zones, étant spécifié que l'admission s'effectue dans la première zone et que les esters d'acides carboxyliques sont prélevés dans la seconde zone.